(19) Europäisches Patentamt
European Patent Office
Office européen des brevets

(11) Veröffentlichungsnummer: **0 374 414**
**A2**

# (12) EUROPÄISCHE PATENTANMELDUNG

(21) Anmeldenummer: **89119447.4**

(22) Anmeldetag: **20.10.89**

(51) Int. Cl.[5]: **C11D 1/72, C11D 1/722, C11D 3/37, C11D 17/00**

(30) Priorität: **22.12.88 DE 3843224**

(43) Veröffentlichungstag der Anmeldung: **27.06.90 Patentblatt 90/26**

(84) Benannte Vertragsstaaten: **AT BE CH DE ES FR GB GR IT LI LU NL SE**

(71) Anmelder: **HÜLS AKTIENGESELLSCHAFT**
**Patentabteilung / PB 15 - Postfach 13 20**
**D-4370 Marl 1(DE)**

(72) Erfinder: **Ruback, Wulf, Dr.**
**Moltkestrasse 15 a**
**D-4350 Recklinghausen(DE)**

## (54) Verdickermischungen für Tenside und Tensidsysteme.

(57) Um optimale anwendungstechnische Eigenschaften wäßriger Tensidlösungen zu erreichen, werden diesen Lösungen Verdickungsmittel zugesetzt. Den bekannten Verdickungsmittel müssen in hohen Konzentrationen den Tensidsystemen zugesetzt werden, die zum Beispiel bei Elektrolyt-Verdickungsmitteln so hoch liegen können, daß Hautirritationen auftreten können.

Die neuen Verdickermischungen sollen diese Nachteile nicht aufweisen.

Hierzu werden Verdickungsmittel für Tenside und Tensidsysteme vorgeschlagen, die aus einer Mischung eins nicht-ionischen Tensides mit einem HLB-Wert von 4 bis 11 mit einem Polymeren bestehen. Als nicht-ionisches Tensid wird eine Verbindung der allgemeinen Formel $R\text{-}(BO)_e(CH_2CH_2O)_f\text{-}X$ und als Polymer eine Verbindung der allgemeinen Formel $_x[R^1\text{-}(CH_2CH_2O)_b\ (BO)_a]\text{-}A\text{-}[(BO)_c\ (CH_2CH_2O)_d\text{-}R^2]_y$ verwendet. Diese Mischungen weisen die oben genannten Nachteile nicht auf.

Es tritt ein synergistischer Verdickungseffekt auf.

Diese Verdickermischungen werden nur in Konzentrationen von 0,05 bis 10 % in Tensid und Tensidsystemen angewendet.

EP 0 374 414 A2

## Verdickermischungen für Tenside und Tensidsysteme

Für optimale anwendungstechnische Eigenschaften wäßriger Lösungen, Emulsionen oder Suspensionen von Tensiden ist deren Viskosität von besonderer Bedeutung. Aus diesem Grunde werden solche Mischungen oft auf höhere Viskositäten eingestellt. Dadurch wird die Handhabung zum Beispiel eines Haarshampoos erleichtert. Es läßt sich einfach dosieren und leichter anwenden, da es sich nur langsam auf der Hand verteilt und nicht von den Haaren fließt. Darüber hinaus bewirkt eine erhöhte Viskosität eine verbesserte Lagerstabilität von Formulierungen, die unlösliche Bestandteile, wie z. B. Buildersubstanzen, Perlglanzpigmente oder Antischuppenmittel, enthalten.

Es sind eine Vielzahl von Verdickungsmitteln bekannt, mit denen sich die Viskosität wäßriger Tensidformulierungen erhöhen läßt. So kann z. B. Kochsalz zur Verdickung von Alkylethersulfaten verwendet werden. Weitere Verdickungsmittel sind Celluloseether, Fettsäurealkanolamide, N-Alkyl-Fettsäureglucamide (DE-OS 37 11 776), Acetamidocarboxylate (US-PS 4 704 226), Polyethylenglykoldistearat, Propandiolpolyoxyethylenetherdifettsäureester (DE-OS 32 39 564), Alkylpolyethylenglykolether-Fettsäureester (DE-OS 35 41 813), Polyethylenglykoldialkylether (DE-OS 35 51 535) oder auch Polyester aus Polyalkylenoxid und dimerisierten Fettsäuren (DE-OS 36 00 263).

Ferner sind Alkylether oder -ester von polyoxalkylierten Polyhydroxyverbindungen für die Verdickung wäßriger Tensidsysteme bekannt (EP-A 0 094 720).

Ein gravierender Nachteil dieser Verdickungsmittel besteht darin, daß sie zur Einstellung der gewünschten Viskosität in hohen Konzentrationen eingesetzt werden müssen. Dies ist auch in solchen Formulierungen der Fall, in denen Elektrolyte als Co-Verdicker verwendet werden können; man benötigt hier ebenfalls so große Mengen Verdikkungsmittel, die ökonomisch nicht vertretbar sind. Des weiteren stehen Elektrolyte, sofern sie in höheren Konzentrationen angewendet werden, in dem Verdacht, Hautirritationen zu erzeugen; somit ist es erforderlich, deren Gehalt möglichst niedrig zu halten.

Dieser Erfindung liegt daher die Aufgabe zugrunde, Verdickungsmittel für Tenside aufzufinden, die in niedrigen Konzentrationen und eventuell mit geringem Elektrolytanteil eine hohe Verdickungswirkung bei einem breiten Spektrum von Tensiden zeigen.

Diese Aufgabe wurde erfindungsgemäß dadurch gelöst, daß man eine Mischung aus nicht-ionischem Tensid mit einem HLB-Wert von 4 bis 11 und einem Polymeren der allgemeinen Formel I

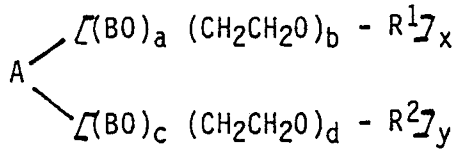

(I)

mit der Bedeutung für

A = Grundkörper einer Verbindung mit 2 bis 24 Aktiv-H-Funktionen,

BO = Oxyalkylengruppe mit 3 bis 4 C-Atomen,

$R^1$, $R^2$ = Wasserstoff oder $C_8$-$C_{24}$-Alkyl-, Alkenyl- oder Acylgruppe,

a, c = ganze Zahlen zwischen 0 und 30,

b, d = ganze Zahlen zwischen 10 und 100 und

x, y = ganze Zahlen von 0 bis 24 mit der Maßgabe, daß die Summe 2 bis 24 beträgt,

als Verdickungsmittel in einer Konzentration von 0,05 bis 10 % einsetzt.

Gegenstand der Erfindung ist daher ein Verdickungsmittel, welches dadurch gekennzeichnet ist, daß man eine Mischung eines nicht-ionischen Tensids mit einem HLB-Wert von 4 bis 11 mit einem Polymeren in einem Gewichtsverhältnis von 10 : 1 bis 1 : 10 verwendet.

Es wurde überraschenderweise gefunden, daß eine Mischung bestehend aus einem nicht-ionischen Tensid mit einem HLB-Wert von 4 bis 11 im Zusammenhang mit einem Polymeren der Formel 1, hervorragende Verdikkungseigenschaften für Tenside und Tensidsysteme aufweist.

Weiterhin wurde gefunden, daß diese leicht verdickend wirkenden, nicht-ionischen Tenside in Mischung mit den Polymeren einen außergewöhnlich hohen synergistischen Verdickungseffekt aufweisen, so daß die Einsatzkonzentration des Verdickungsmittels zur Einstellung der gewünschten Viskosität nur zwischen 0,05 und 10 %, vorzugsweise 0,1 und 5 %, und insbesondere zwischen 0,25 und 4 % liegt.

Das Gewichtsverhältnis von Polymer-Verdickungsmittel zu nicht-ionischem Tensid liegt bei den erfindungsgemäßen Mischungen zwischen 10 : 1 und 1 : 10, bevorzugt zwischen 6 : 1 und 1 : 8, und

insbesondere zwischen 2 : 1 und 1 : 6.

Als nicht-ionische Tenside können hierbei Verbindungen verwendet werden, die der Formel II

$R - (BO)_e (CH_2CH_2O)_f - X$, (II)

mit R = Alkyl, Alkenyl oder Alkylaryl-Rest mit jeweils 6 bis 22 C-Atomen, insbesondere 8 bis 18 C-Atomen,
BO = Oxyalkylengruppe mit 3 bis 4 C-Atomen,
X = H oder Alkylrest mit 1 bis 4 C-Atomen,
e = 0 bis 6 und
f = 1 bis 15,
entsprechen, mit der Maßgabe, daß der HLB-Wert 4 bis 11, vorzugsweise 4,5 bis 10, und insbesondere 5 bis 9 ist. Bevorzugt sind Verdickungsmittel mit e = 0 und f = 1 bis 6.

Als erfindungsgemäße Polymere gelangen Verbindungen der Formel I

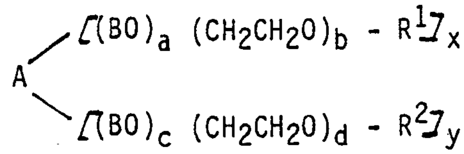

(I)

mit der Bedeutung für
A = Grundkörper einer Verbindung mit 2 bis 24-, vorzugsweise 2 bis 14, insbesondere 2 bis 8-Aktiv-H-Funktionen,
BO = Oxyalkylengruppe mit 3 bis 4 C-Atomen,
$R^1$, $R^2$ = Wasserstoff oder $C_8$-$C_{24}$-Alkyl-, Alkenyl- oder Acylgruppe,
a, c = ganze Zahlen zwischen 0 und 30,
b, d = ganze Zahlen zwischen 10 und 100 und
x, y = ganze Zahlen von 0 bis 24 mit der Maßgabe, daß die Summe 2 bis 24 beträgt,
zum Einsatz.

Beispiele für erfindungsgemäße Polymere sind Ethylenglykol, Terephthalsäure, Ethylendiamin, Triethanolamin, Glycerin, Diethylentriamin, Pyromellithsäure, Butan-1,2,3,4-tetracarbonsäure, Pentaerythrit, Erythrit, Sorbit, Glucose, Saccharose, Maltose Polyvinylalkohol (u = 2 -24) oder Polyacrylsäure (u = 2 - 24).

Mit den erfindungsgemäßen Verdickermischungen lassen sich klare viskose bis hochviskose Einstellungen der gängigen nicht-ionischen und/oder anionischen und/oder amphoteren Tenside in einfacher Weise herstellen.

Diese können pharmazeutische, kosmetische, Haushalts- oder auch technische Präparate sein. Dabei ist auch die Kombination der erfindungsgemäßen Produkte mit anderen üblichen Bestandteilen dieser Präparate möglich. So können sie beispielsweise in Haarpflegemittel, Hautreinigungsmittel, wie beispielsweise Schaum- und Duschbadpräparate, Zahnreinigungsmittel, Haarverformungsmittel, Salbe, Geschirrspülmittel, Waschmittel, Kraftfahrzeugreinigungsmittel, Haushaltsreinigungsmittel und andere technische Reinigungspräparate eingearbeitet werden. Die Einarbeitung dieser synergistischen Mischung in die zu verdickenden Lösungen, Suspensionen oder Emulsionen erfolgt in an sich bekannter Weise durch Auflösen des Verdickungsmittels, eventuell unter Erwärmen, in Wasser bzw. in einer wasserhaltigen Phase.

In einigen anionischen Tensidsystemen, wie z. B. alkylethersulfathaltigen Formulierungen, beobachtet man dabei einen ausgesprochenen Synergismus mit Elektrolyten, wie z. B. Kochsalz oder Bittersalz, wodurch die Gesamtmenge des zur Einstellung einer bestimmten Viskosität benötigten Viskositätsreglers weiter reduziert wird bzw. die an sich schon überraschend guten Verdickungseigenschaften der erfindungsgemäßen Produkte dadurch noch verstärkt werden.

Zusätzlich zu den rheologischen Eigenschaften verbessern die erfindungsgemäßen Viskositätsregler die Schäumeigenschaften der sie enthaltenden Formulierungen. Bei entsprechenden Haarpflegepräparaten beobachtet man einen Konditioniereffekt, d. h. die Kämmbarkeit und Fülle des Haars wird verbessert.

In den nachfolgenden Beispielen werden die erfindungsgemäßen Verdikkermischungen näher erläutert.

Die Viskositäten werden mit Hilfe eines rechnergesteuerten Rotationsviskosimeters der Firma Haake (Typ: RV 12) bei einer Temperatur von 25 °C gemessen.

| Verwendete Produktnamen und Abkürzungen: | |
|---|---|
| Antil 141 solid (Fa. Goldschmidt) | - Propylenglykol-PEG55-dioleat |
| Dehydol LS 3 DEO (Fa. Henkel) | - Fettalkoholpolyglykolether (HLB-Wert ca. 8,8) |
| DIONIL® OC | - Oleylamidopolyglykolether (Rückfettungskomponente) |
| MARLINAT® CM 105 | - Fettalkoholpolyglykoletheressigsäure-Na-Salz (22 % Akt.-Subst.) |
| MARLINAT® 24/70 | - Fettalkoholethersulfat (70 % Akt.-Subst.) |
| MARLINAT® 242/70 | |
| MARLIPAL® 0 13/20 | - Oxoalkoholpolyglykolether (HLB-Wert = 6,1) |
| MARLIPAL® 24/20 | - Fettalkoholpolyglykolether (HLB-Wert = 6,2) |
| PE-200 ST | - Pentaerythritpolyglykolether(200 EO)-tetrastearat |
| SERDET® DFK 30 | - Fettalkoholethersulfat (28 % Aktivsubstanz) |

Beispiel 1

Tensid: MARLINAT® 24/70 (Fettalkoholethersulfat)
Konzentration: 5 % Aktivsubstanz, 2,5 % Kochsalz
Verdickungsmittel:
I) Umsetzungsprodukt aus Pyromellithsäuredianhydrid und Oleylalkoholethoxilat (50 mol EO), (Molverhältnis 1 : 1,1)
II) MARLIPAL® 0 13/20, HLB-Wert = 6,1
Konzentration: I + II = 1 %

| Verhältnis I : II | Viskosität (D = 2 $sec^{-1}$) [mPas] |
|---|---|
| 1 : 0 (reines I) | < 10 |
| 1 : 1 | 230 |
| 1 : 2 | 2 300 |
| 1 : 3 | 2 900 |
| 1 : 4 | 3 500 |
| 1 : 6 | 2 800 |
| 1 : 10 | 1 800 |
| 0 : 1 (reines II) | 420 |
| ohne I oder II | < 10 |

Beispiel 2

Tensid: MARLINAT® 24/70
Konzentration: 15 % Aktivsubstanz, 2,5 % Kochsalz
Verdickungsmittel:
I) Umsetzungsprodukt aus Pyromellithsäuredianhydrid und Oleylalkoholethoxilat (50 mol EO) Molverhältnis 1 : 1,2
II) MARLIPAL® 24/20, HLB-Wert = 6,2
Konzentration: I + II = 2 %

| Verhältnis I : II | Viskosität (D = 2 $sec^{-1}$) [mPas] |
|---|---|
| 1 : 0 | 12 460 |
| 1 : 1 | 57 000 |
| 1 : 3 | 55 000 |
| 1 : 7 | 39 000 |
| 0 : 1 | 4 390 |
| ohne I oder II | 180 |

Beispiel 3

Tensid: MARLIPAL® 24/20
Konzentration: 5 % Aktivsubstanz, 2,5 % Kochsalz
Verdickungsmittel:
I) wie in Beispiel 2
II) Dehydol LS 3 Deo, HLB-Wert ca. 8,8
Konzentration: I + II = 2 %

| Verhältnis I : II | Viskosität (D = 2 $sec^{-1}$) [mPas] |
|---|---|
| 1 : 0 | 20 |
| 1 : 1 | 3 240 |
| 1 : 3 | 11 300 |
| 0 : 1 | 1 900 |
| ohne I oder II | < 10 |

Beispiel 4

Tensid: MARLINAT® 242/70
Konzentration: 15 % Aktivsubstanz, 2 % Kochsalz
Verdickungsmittel:
I) PE-200 ST
II) MARLIPAL® 24/20, HLB-Wert = 6,2
Konzentration: I + II = 1,5 %

| Verhältnis I : II | Viskosität (D = 2 $sec^{-1}$) [mPas] |
|---|---|
| 1 : 0 | 46 200 |
| 1 : 1 | 92 600 |
| 0 : 1 | 4 170 |
| ohne I oder II | 10 |

Beispiel 5

| Schaumbad mit 16 % Aktivsubstanz | | |
|---|---|---|
| Rezeptur: | SERDET® DFK 30 | 30,0 % |
| | MARLINAT® CM 105 | 35,7 % |
| | DIONIL® OC | 5,0 % |
| | Verdickungsmittel | 2,0 % |
| | Parfum | 0,3 % |
| | Farbstoff | 0,3 % |
| | Konservierungsmittel, Wasser | ad 100 % |

| Verdickungsmittel | Viskosität (D = 2 $sec^{-1}$) [mPas] |
|---|---|
| ohne | 60 |
| NaCl | 4 500 |
| MARLIPAL® 24/20 | 2 600 |
| Antil 141 | 9 010 |
| 1 Teil Antil 141<br>3 Teile MARLIPAL® 24/20 | 10<br>215 |
| PE-200 ST | 8 750 |
| 1 Teil PE-200 ST<br>3 Teile MARLIPAL® 24/20 | 22<br>600 |

Beispiel 6

| Haarshampoo mit 15 % Aktivsubstanz | | |
|---|---|---|
| Rezeptur: | SERDET® DFK 30 | 26,5 % |
| | MARLINAT® CM 105 | 36,0 % |
| | DIONIL® OC | 2,0 % |
| | 1,2-Propylenglykol | 1,5 % |
| | Natriumchlorid | 3,0 % |
| | Verdickungsmittel | 1,0 % |
| | Parfum | 0,3 % |
| | Farbstoff | 0,3 % |
| | Konservierungsmittel, Wasser | ad 100 % |

| Verdickungsmittel | Viskosität (D = 2 $sec^{-1}$) [mPas] |
|---|---|
| ohne<br>MARLIPAL® 24/20<br>PE-200 ST | 10<br>130<br>240 |
| 1 Teil PE-200 ST<br>3 Teile MARLIPAL® 24/20 | 3<br>120 |

## Ansprüche

1. Verdickungsmittel für Tenside und Tensidsysteme,
dadurch gekennzeichnet,
daß man eine Mischung eines nicht-ionischen Tensids mit einem HLB-Wert von 4 bis 11 mit einem Polymeren in einem Gewichtsverhältnis von 10 : 1 bis 1 : 10 verwendet.

2. Verdickungsmittel nach Anspruch 1,
dadurch gekennzeichnet,
daß als nicht-ionisches Tensid Verbindungen der allgemeinen Formel II
$R - (BO)_e (CH_2CH_2O)_f - X$, (II)
mit R = Alkyl, Alkenyl oder Alkylaryl-Rest mit jeweils 6 bis 22 C-Atomen,
BO = Oxyalkylengruppe mit 3 bis 4 C-Atomen,
X = H oder Alkylrest mit 1 bis 4 C-Atomen,
e = 0 bis 6 und
f = 1 bis 15,
eingesetzt werden.

3. Verdickungsmittel nach Anspruch 2,
dadurch gekennzeichnet,
daß in Formel II e = 0 und f = 1 bis 6 ist.

4. Verdickungsmittel nach Anspruch 1,
dadurch gekennzeichnet,
daß das Polymer eine Verbindung der allgemeinen Formel I

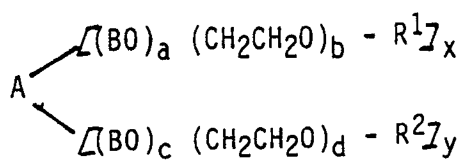

(I)

mit A = Grundkörper einer Verbindung mit 2 bis 24 AktivH-Funktionen,
BO = Oxyalkylengruppe mit 3 bis 4 C-Atomen,
$R_1$, $R_2$ = Wasserstoff oder $C_8$-$C_{24}$-Alkyl-, Alkenyl- oder Acylgruppe,
a, c = ganze Zahlen zwischen 0 und 30,
b, d = ganze Zahlen zwischen 10 und 100 und
x, y = ganze Zahlen von 0 bis 24 mit der Maßgabe, daß die Summe 2 bis 24 beträgt,
ist.

5. Verdickungsmittel nach Anspruch 1,
dadurch gekennzeichnet,
daß die Verdickungsmittel-Konzentration 0,05 bis 10 % beträgt.